# EUROPEAN PATENT APPLICATION

(11) **EP 4 046 634 A1**
(43) Date of publication of application: **24.08.2022**
(21) Application number: 21305196.4
(22) Date of filing: 17.02.2021
(51) Int. Cl.: A61K 31/365, A61K 31/00, A61K 31/352, A61K 31/407, A61K 31/4184, A61K 31/5415, A61K 31/7084, A61K 45/06, A61K 47/00, A61P 31/12, A61P 31/14, A61P 35/00

(54) **SERCA2 MODULATORS AND THEIR THERAPEUTIC USES**

(71) Applicant: Centre national de la recherche scientifique, 75016 Paris (FR); Université de Montpellier, 34090 Montpellier (FR)
(72) Inventor: LAGUETTE, Nadine, 34830 CLAPIERS (FR); VILA, Isabelle Krystel, 30610 LOGRIAN FLORIAN (FR); STEER, Alizée Julie, 34070 MONTPELLIER (FR)
(74) Representative: Lavoix

(57) **Abstract**

The invention relates to the use of SERCA2 (Sarco/Endoplasmic Reticulum Ca²⁺-ATPase 2) modulator in treating an inflammation related-pathology; and to products comprising at least one inhibitor of SERCA2 according to the invention, and at least one activator of STING, as combination products for a simultaneous, separate or sequential use in the treatment and/or prevention of a viral infection or a cancer.

## Description

The present invention relates to the use of a SERCA2 modulator for treating an inflammation related-pathology. Especially, the invention relates to the use of at least one SERCA2 inhibitor for treating viral infections or cancer, notably by boosting cytokines production and immune cells priming; or to the use of at least one SERCA2 activator to decrease inflammation associated with STING.

The STimulator of INterferon Genes (STING) protein is an endoplasmic reticulum (ER) resident protein that plays a central role in innate immunity. Indeed, STING is an adaptor protein that orchestrates transcriptional activation of type I interferons and inflammatory cytokines in the presence of pathological nucleic acid species. STING activation relies on the detection of dsDNA, ssDNA, or RNA:DNA hybrids by the cyclic GMP-AMP synthetase (cGAS) pathogen recognition receptor. Association of cGAS with these nucleic acid species in the cytosol found to lead to cGAS-dependent synthesis of cyclic GMP-AMP (cGAMP). Interaction of cGAMP with STING activates a pathway that finally leads to the transcription of inflammatory cytokines and type I interferons.

Recent research effort has aimed to identify means to modulate the cGAS-STING pathway. In particular STING-targeting immunotherapies aim to activate the inflammatory responses, in immunosuppressed contexts. For example, Demaria *et al* (Demaria, O. et al. STING activation of tumor endothelial cells initiates spontaneous and therapeutic antitumor immunity. Proc. Natl. Acad. Sci. U. S. A. 112, 15408-15413 (2015)) have shown that activation of STING by intra-tumoral injection of cGAMP potentiates the anti-tumor CD8 T cell responses in mouse models of melanoma or colon cancer leading to control of tumor growth. Tang *et al* (Tang, C. H. A. et al. Agonist-mediated activation of STING induces apoptosis in malignant B cells. Cancer Res. 76, 2137-2152 (2016)) have presented evidence that activation and/or overexpression of STING can trigger apoptosis in tumor cells, inducing the release of tumor antigens and therefore promoting recognition by the immune system.

To date, all the molecules undergoing clinical trials for modulating the activation of STING directly act on this protein. The side effects, such as metabolic modulation associated with direct activation of STING, are just starting to be characterized. Thus, there is a need for reducing these side effects and improving the efficacy of STING agonists. Especially, a subset of patients is refractory to conventional immunotherapies. There is thus a need for therapeutic agents that could overcome these therapeutic resistances.

To the contrary, several autoimmune or auto-inflammatory pathologies present with chronic STING activation. Such diseases include, but are not restricted to, Interferonopathies or STING-Associated Vasculopathy with onset in Infancy (SAVI). The former generally result from accumulation of pathological nucleic acids that activate STING while the latter results from expression of constitutively active STING alleles. These pathologies are classically treated with inhibitors of the Janus kinase (JAK), blocking the type I IFN-signalling. Yet, these therapeutic approaches cause side effects, including increased susceptibility to pathogen infection, calling for alternative therapeutic strategies.

There is thus a need for novel and efficient therapies involving the STING pathway. There is also a need for novel and efficient drugs useful for preventing and/or treating cancer-related inflammation, while mitigating side effects.

The present invention solves these needs.

Indeed, the inventors have surprisingly discovered that the Sarco/Endoplasmic Reticulum Ca²⁺-ATPase 2 (SERCA2) is a binding partner of STING. This new pathway is of major interest for treating pathologies involving STING activation, as well as for treating pathologies involving STING inhibition.
More specifically, the inventors sought to identify novel mechanisms involved in the regulation of STING. For this purpose, they immunopurified STING and protein partners, and used Mass Spectrometry analysis to reveal novel interactions. Amongst identified partners, they recovered SERCA2 as a binding partner of STING and this interaction was checked by Western Blot on immuno-precipitated Flag-tagged STING (data not shown).
As shown in the example, the inventors then evidenced that the inhibition of SERCA2 increased the inflammatory response in wild-type cells, boosting the effect of a known STING agonist, as well as in a model of chronic STING activation.
In conclusion, they showed that SERCA2 is an inhibitor of STING, and SERCA2 inhibitors potentiate the interferon response both in chronic models of STING activation and upon acute stimulations.

Thus, modulating SERCA2 could improve the response to immunotherapies, while allowing the reduction of the doses of STING agonist to be administered.

As a consequence, the present invention proposes the use of a SERCA2 modulator for treating an inflammation related-pathology.

In a first embodiment, the present invention relates to the use of at least one SERCA2 inhibitor for treating viral infections or cancer.
In the context of cancer, the inflammatory state of tumors is a determinant component of their outcome, and inflammation plays a crucial role at all stages of tumorigenesis, from the appearance of neoplasic lesions to metastatic spread. This inflammatory state also determines the immunogenicity of tumors and their response to immunotherapy, distinguishing "hot" tumors from "cold" tumors.
Activation of the cGAS and STING-based pro-inflammatory signaling pathway in tumor cells has been documented in recent years, and in specific cases, to inhibit tumor progression by a number of mechanisms, including the promotion of tumor recognition by the immune system, leading to their destruction. In addition, activation of the STING pathway has been reported to improve the response to immunotherapy. These aspects are not observed in all cancer cases; it depends in particular on the grade and the origin of the tumor.
To date, approaches to activate STING are under development. The inventors offer an alternative or combinatorial approach to activate STING by using at least one SERCA2 inhibitor.
Thus, in the context of a cancer for which an inflammatory response is required, the activation of STING and the interferon response may be induced by a SERCA2 inhibitor. Such a cancer may be one presenting a low immunogenicity, or a cancer where the response to immunotherapy and/or radiotherapy could be reinforced.
In the case of a viral infection, the interferon response is of major impact. By using an inhibitor of SERCA2 according to the invention, STING activation could be reinforced, leading to interferon response and to a faster elimination of the virus.

In a second embodiment, the present invention relates to the use of at least one SERCA2 activator to decrease inflammation associated with STING. This could be of interest for treating chronic infectious diseases that include chronic STING activation, as well as chronic cancer-related inflammation.

The invention will be better understood in view of the following.

SERCA2 (Sarco/Endoplasmic Reticulum Ca²⁺-ATPase 2), also called ATP2A2, is one of the 3 SERCA proteins that are responsible for creating a Ca²⁺ gradient from the cytosol to the lumen of the ER and as such is essential to the maintenance of Ca²⁺ homeostasis in cells. Interestingly, recent studies have documented that calcium (Ca²⁺) fluxes can influence the activation of STING. In absence of stimulation, the stromal interaction molecule 1 (STIM1) transmembrane calcium sensor of the ER interacts with STING, preventing its interaction with TBK1. In the presence of Ca²⁺, STIM1 promotes anchoring of STING in the ER membrane. When the Ca²⁺ of the ER is exhausted, STIM1 releases STING, allowing its translocation to the Golgi upon activation. Thus, Ca²⁺ fluxes are central to STING regulation. The amino acid sequence of human SERCA2 can be found in Uniprot under accession number P16615.

The present invention relates to the use of a SERCA2 modulator for treating an inflammation related-pathology.

By "modulator", it is meant a compound which is an inhibitor or an activator of SERCA2. The modulator is a ligand of SERCA2, i.e. it binds to SERCA2.

By "SERCA2 inhibitor" or "inhibitor of SERCA2", it is meant a compound that reversibly or irreversibly binds to SERCA2 and decreases its activity. A reversible binding is a noncovalent interaction between the inhibitor and SERCA2. An irreversible binding is a covalent interaction between the inhibitor and SERCA2. The inhibitor may be competitive or non-competitive, preferably the inhibitor is non-competitive. Preferably, the inhibitor is specific of SERCA2. By "specific" it is meant that the inhibitor has an IC50 *in vitro* for SERCA2 (for calcium entry) of less than 2.5 µM, preferably of less than 1 µM. Preferably, such an inhibitor is a small molecule.
Examples of inhibitors of SERCA2 are notably already commercially available, as it is the case for thapsigargin or cyclopiazonic acid (CPA).

Thapsigargin (IUPAC name: (3S,3aR,4S,6S,6aR,7S,8S,9bS)-6-(Acetyloxy)-4-(butyryloxy)-3,3a-dihydroxy-3,6,9-trimethyl-8-{[(2Z)-2-methylbut-2-enoyl]oxy}-2-oxo-2,3,3a,4,5,6,6a,7,8,9b-decahydroazuleno[4,5-b]furan-7-yl octanoate) is the compound of formula (I) below: 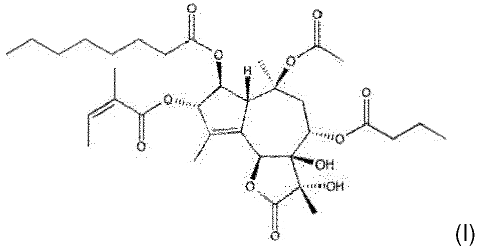 Thapsigargin is an irreversible SERCA2 inhibitor. It is a specific SERCA2 inhibitor, i.e. it shows an IC50 *in vitro* for SERCA2 (for calcium entry) of less than 15 nM.

CPA (IUPAC name: (6aR,11aS,11bR)-10-Acetyl-11-hydroxy-7,7-dimethyl-2,6,6a,7,11a,11b-hexahydro-9H-pyrrolo[1',2':2,3]isoindolo[4,5,6-cd]indol-9-one) is the compound of formula (II) below: 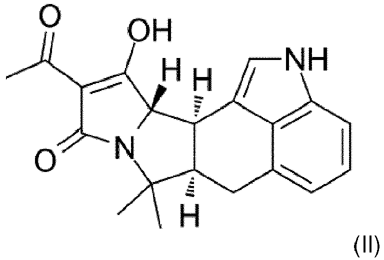 It is a specific SERCA2 inhibitor, i.e. it shows an IC50 *in vitro* for SERCA2 (for calcium entry) of less than 2500 nM.

By "SERCA2 activator" or "activator of SERCA2", it is meant a compound that reversibly or irreversibly binds to SERCA2 and increases its activity. A reversible binding is a noncovalent interaction between the inhibitor and SERCA2. An irreversible binding is a covalent interaction between the activator and SERCA2. Preferably the activator is allosteric. Preferably the activator is a small molecule.
Examples of activators of SERCA2 are notably already commercially available, as it is the case for CDN-1163.
CDN-1163 (IUPAC name: N-(2-methylquinolin-8-yl)-4-propan-2-yloxybenzamide) is the compound of formula (III) below:

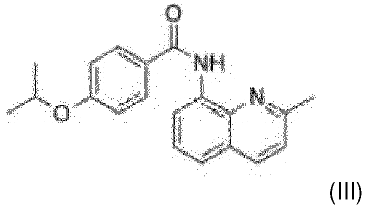

In order to determine whether a test compound is a SERCA2 inhibitor or a SERCA2 activator, the following protocol may be used for the measurement of calcium fluxes:
To measure the amount of calcium stored in endoplasmic reticulum after a test compound treatment, fluorescence will be measured in a medium lacking Ca2+ and Mg2+ in 96 well plates.
Cells will be loaded with Fluo-4 acetoxymethyl (Fluo-4 AM; Thermofisher) at 37°C for 30 minutes in the dark and then incubated with different concentrations of a test compound (for example 500 nM of thapsigargin) or control (DMSO). Fluo-4 AM is a labeled calcium indicator which is a molecule that exhibits an increase in fluorescence upon binding Ca²⁺.
Changes in fluorescence (485/530 nm) will be measured over a 5-minute period using a microplate reader. Data will be expressed as fluorescence units in test compound-treated cells minus the fluorescence units in the control cells.
If the fluorescence decreases with increasing concentrations of the test compound, then the test compound is a SERCA2 activator.
If the fluorescence increases with increasing concentrations of the test compound, then the test compound is a SERCA2 inhibitor.

The inhibitor of SERCA2 of the present invention is able to inhibit SERCA2 activity, thereby leading to STING activation, and to interferon response.
The use of said inhibitor according to the invention is for treating viral infections or cancer.

The activator of SERCA2 of the present invention is able to decrease inflammation associated with STING. The use of said activator according to the invention is for treating chronic infectious diseases that include chronic STING activation, as well as cancer-related inflammation. Preferably, cancer-related inflammation involves the SERCA2-STING pathway.

By "inflammation", it is meant the inflammation that is triggered by activation of STING and that leads to type I interferon production (interferon response). Said inflammation is typically triggered in the presence of pathological immune-stimulatory nucleic acids that can either be endogenous (i.e. resulting from mitochondrial or nuclear damage for example) or exogenous (i.e. pathogen-derived). The inflammation may be acute or chronic. It includes cancer-related inflammation, but also the inflammation component of chronic inflammatory diseases such as lupus (i.e. systemic lupus erythematosus), Aicardi-Goutieres syndrome, obesity, inflammatory bowel disease (IBD) or arthritis.
By "cancer-related inflammation", it is meant the inflammation which is due to cancer, or the inflammation which causes cancer. Said inflammation is characterized by the presence of pro-inflammatory cytokines (such as CxCL 10 and ISG15) and of type I interferon.
Particularly, the activator of SERCA2 of the invention is used for decreasing and/or inhibiting the production of type I interferon and/or pro-inflammatory cytokines (such as CxCL 10 and ISG15) notably in cancer cells, via interaction with the SERCA2-STING pathway.
Particularly, the inhibitor of SERCA2 of the invention is used for increasing and/or activating the production of type I interferon and/or pro-inflammatory cytokines (such as CxCL10 and ISG15), via interaction with the SERCA2-STING pathway.

By "treatment" of a given disease, it is meant the curative treatment of said disease. A curative treatment is defined as a treatment that completely treats (cures) or partially treats the disease.
In the case of a treatment of cancer, it is defined as a treatment that completely treats (cures) or partially treats cancer, i.e. induces tumor growth stabilization, retardation or regression.
The "subject" refers to any subject and typically designates a patient, preferably a subject afflicted by a DNA virus, such as Herpes Simplex Viruses, Varicella Zooster Virus; or a subject afflicted by a RNA virus, such as Retroviruses including lentiviruses such as Human Immunodeficiency Type I and II viruses; or a subject afflicted by an inflammatory disease, or undergoing a treatment of cancer such as immunotherapy, chemotherapy and/or radiotherapy. The subject may be afflicted by a cancer, or by a chronic inflammatory disease, notably chosen from lupus (i.e. systemic lupus erythematosus), Aicardi-Goutieres syndrome, obesity, inflammatory bowel disease (IBD) and arthritis. In any case, the subject is preferably a vertebrate, more preferably a mammal, even more preferably a human being.

By "cancer", it is meant any type of cancer. The cancer may be solid or non-solid, and may be for example selected from a colon cancer, a colorectal cancer, a melanoma, a bone cancer, a breast cancer, a thyroid cancer, a prostate cancer, an ovarian cancer, a lung cancer, a pancreatic cancer, a glioma, a cervical cancer, an endometrial cancer, a head and neck cancer, a liver cancer, a bladder cancer, a renal cancer, a skin cancer, a stomach cancer, a testis cancer, an urothelial cancer or an adrenocortical carcinoma, leukemia but also non solid cancers such as lymphoma.
Preferably, the cancer is a pancreatic cancer, a breast cancer, a prostate cancer, a lung cancer, a liver cancer, a bone cancer, a bladder cancer or a leukemia.

The modulator of the invention is preferably administered at a therapeutically effective amount or dose. As used herein, "a therapeutically effective amount or dose" refers to an amount of the modulator of the invention which prevents, removes, slows down the disease, or reduces or delays one or several symptoms or disorders caused by or associated with said disease in the subject, preferably a human being. The effective amount, and more generally the dosage regimen, of the modulator of the invention and pharmaceutical compositions thereof may be determined and adapted by the one skilled in the art. An effective dose can be determined by the use of conventional techniques and by observing results obtained under analogous circumstances. The therapeutically effective dose of the modulator of the invention will vary depending on the disease to be treated or prevented, its gravity, the route of administration, any co-therapy involved, the patient's age, weight, general medical condition, medical history, etc.
Typically, the amount of the modulator to be administered to a patient may range from about 0.01 to 500 mg/kg of body weight for a human patient. In a particular embodiment, the pharmaceutical composition according to the invention comprises 0.01 mg/kg to 300 mg/kg of the modulator of the invention, preferably from 0.01 mg/kg to 3 mg/kg, for instance from 25 to 300 mg/kg.
In a particular aspect, the modulator of the invention can be administered to the subject by intratumoral route, parenteral route, topical route, oral route or intravenous injection. The modulator of the invention may be administered to the subject daily (for example 1, 2, 3, 4, 5, 6 or 7 times a day) during several consecutive days, for example during 2 to 10 consecutive days, preferably from 3 to 6 consecutive days. Said treatment may be repeated during 1, 2, 3, 4, 5, 6 or 7 weeks, or every two or three weeks or every one, two or three months. Alternatively, several treatment cycles can be performed, optionally with a break period between two treatment cycles, for instance of 1, 2, 3, 4 or 5 weeks. The modulator of the invention can for example be administered as a single dose once a week, once every two weeks, or once a month. The treatment may be repeated one or several times per year. Doses are administered at appropriate intervals which can be determined by the skilled person. The amount chosen will depend on multiple factors, including the route of administration, duration of administration, time of administration, the elimination rate of the compound, or of the various products used in combination with said compound, the age, weight and physical condition of the patient and his/her medical history, and any other information known in medicine.

The administration route can be intratumoral, oral, topical or parenteral, typically rectal, sublingual, intranasal, intra-peritoneal (IP), intra-venous (IV), intra-arterial (IA), intramuscular (IM), intra-cerebellar, intrathecal, intratumoral and/or intradermal. The pharmaceutical composition is adapted for one or several of the above-mentioned routes. The pharmaceutical composition is preferably administered by intratumoral route. The pharmaceutical composition may be injected in the tumor (*in situ*) or near the tumor. Said intratumoral route preferably includes the administration of long lasting delivery forms.

The present invention also relates to a composition comprising, in a pharmaceutically acceptable medium, at least one SERCA2 modulator according to the invention. Such a composition comprises a pharmaceutically acceptable medium (or carrier).
The carrier must be "acceptable" in the sense of being compatible with the other ingredients of the formulations and not deleterious to the recipient thereof.
The pharmaceutical composition can be formulated as a long lasting delivery form. Such forms include polymeric nanoparticles and notably PLGA (Poly(lactic-co-glycolic acid)) nanoparticles, hydrogels or any other galenic form usable for intratumor delivery.
The pharmaceutical composition can be formulated as solutions in pharmaceutically compatible solvents or as gels, oils, emulsions, suspensions, or dispersions in suitable pharmaceutical solvents or vehicles, or as pills, tablets, capsules, powders, suppositories, etc. that contain solid vehicles in a way known in the art, possibly through dosage forms or devices providing sustained and/or delayed release. For this type of formulation, an agent such as cellulose, lipids, carbonates or starches are used advantageously.
Agents or vehicles that can be used in the formulations (liquid and/or injectable and/or solid) are excipients or inert vehicles, i.e. pharmaceutically inactive and non-toxic vehicles.
Mention may be made, for example, of saline, physiological, isotonic and/or buffered solutions, compatible with pharmaceutical use and known to those skilled in the art. The compositions may contain one or more agents or vehicles chosen from dispersants, solubilizers, stabilizers, preservatives, etc.
Particular examples are methylcellulose, hydroxymethylcellulose, carboxymethylcellulose, cyclodextrins, polysorbate 80, mannitol, gelatin, lactose, liposomes, vegetable oils or animal, acacia, etc. Preferably, vegetable oils are used.
Formulations of the present invention suitable for oral administration may be in the form of discrete units as capsules, sachets, tablets or lozenges, each containing a predetermined amount of the active ingredient; in the form of a powder or granules; in the form of a solution or a suspension in an aqueous liquid or non-aqueous liquid; or in the form of an oil-in-water emulsion or a water-in-oil emulsion.
Formulations suitable for parenteral administration conveniently comprise a sterile oily or aqueous preparation of the active ingredient which is preferably isotonic with the blood of the recipient. Every such formulation can also contain other pharmaceutically compatible and non-toxic auxiliary agents, such as, e.g. stabilizers, antioxidants, binders, dyes, emulsifiers or flavoring substances.

Especially, the present invention relates to products comprising:
a) At least one inhibitor of SERCA2 according to the invention, and
b) At least one activator of STING,
as combination products for a simultaneous, separate or sequential use in the treatment and/or prevention of a viral infection or a cancer.
It also relates to the use of at least one inhibitor of SERCA2 according to the invention, for preventing and/or treating a viral infection or a cancer in combination or in association with at least one activator of STING.
It further relates to the use of at least one inhibitor of SERCA2 according to the invention, for preventing and/or treating a viral infection or a cancer in a subject treated by at least one activator of STING.
The inhibitor of SERCA2 according to the invention is as described above.
The activator of STING is a compound that reversibly or irreversibly binds to STING and increases its activity. Such an activator may be a small molecule. Preferably, the activator of STING is DMXAA; ADU-S100; ABZI (amidobenzimidazole)-based STING agonists (diABZIs) or G10.

DMXAA, also called acid acetic-4-dimethylxanthenone-6,5, has the following structure : 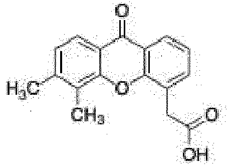 DMXAA is murine specific.

ADU-S100, also called MIW815, is a synthetic cyclic dinucleotide. It has the following structure : 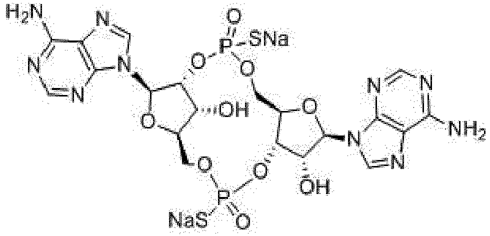 diABZIs are STING agonists sharing ABZI core. A preferred diABZI compound is a potent non-nucleotide STING agonist of the following structure: 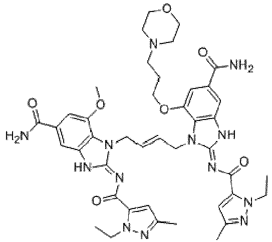 STING agonist-1 (G10) is a novel human-specific STING agonist. It has the following structure:

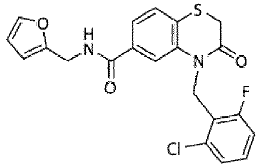

The present invention is illustrated by the following figures:

### Figures

**Figure** 1: **Inhibition of SERCA2 (ATP2A2) increased inflammatory response of WT-MEF cells.**
   WT-MEF cells were cultured 24h prior 2h stimulation with DMXAA (200µM) in combination or not with CPA **(A-C)** or thapsigargin **(D-E).** Graphs present fold increase lfnβ, Cxcl10 and Isg15 mRNA levels, as compared to unstimulated cells. Graphs present mean value from duplicates.
**Figure 2****: Inhibition of SERCA2 (ATP2A2) increases inflammatory response of Trex I KO MEFs.**
   MEF^{Trexl-/-} cells were treated or not with CPA **(A-C)** or thapisgargin **(D-E)** for 2h. Graphs present fold increase lfnβ, Cxcl10 and Isg15 mRNA levels, as compared to unstimulated cells. Graphs present mean value from duplicates (A-C) or average of 2 independent experiments (D-F). Error bars: standard deviation.

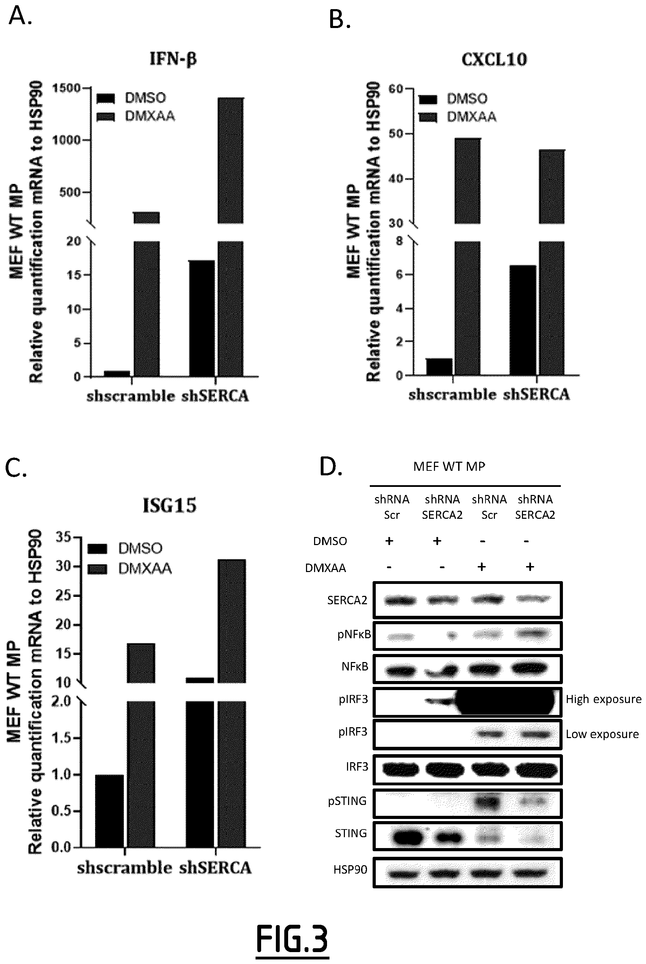

**Figure 3****: Knockdown of SERCA2 (ATP2A2) increases inflammatory responses in WT-MEFs.**
   MEF expressing non-targeting (Scramble) or ATP2A2-targeting shRNAs were stimulated or not with DMXAA (200µM) for 2h. **(A-C)** Graphs present mean fold change lfnβ, Cxcl10 and Isg15 mRNA levels as measured by RT-qPCR. **(D)** WB analysis of whole-cell extracts of cells treated as in (A-C) using indicated antibodies.

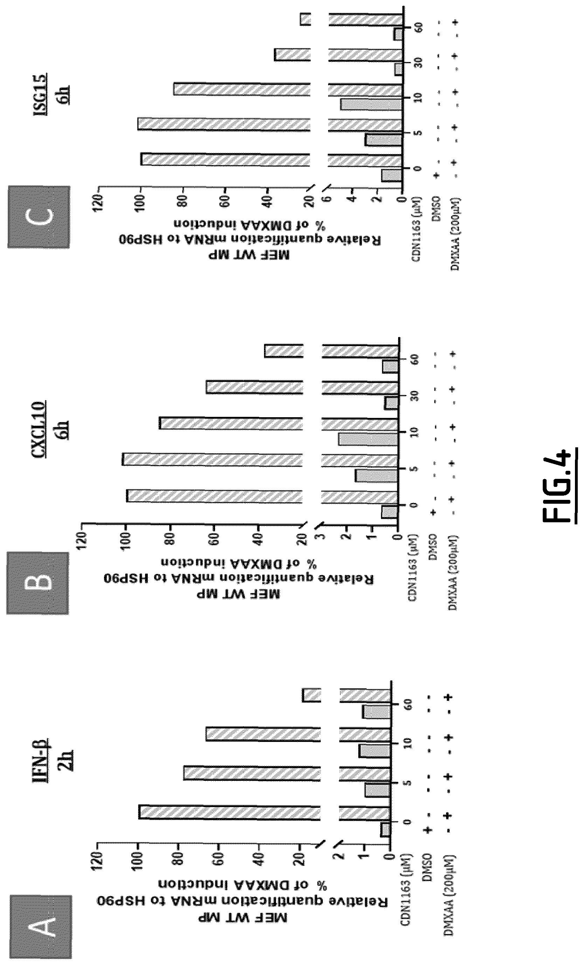

**Figure 4****: Treatment with the CDN-1163 SERCA2 (ATP2A2) allosteric activator decreases inflammatory responses in WT-MEFs.**
   WT-MEF cells were cultured 24h prior 2h stimulation with DMXAA (200µM) in combination or not with CDN-1163 **(A-C).** Graphs present fold increase lfnβ, Cxcl10 and lsg 15 mRNA levels, as compared to unstimulated cells. Graphs present mean value from duplicates.

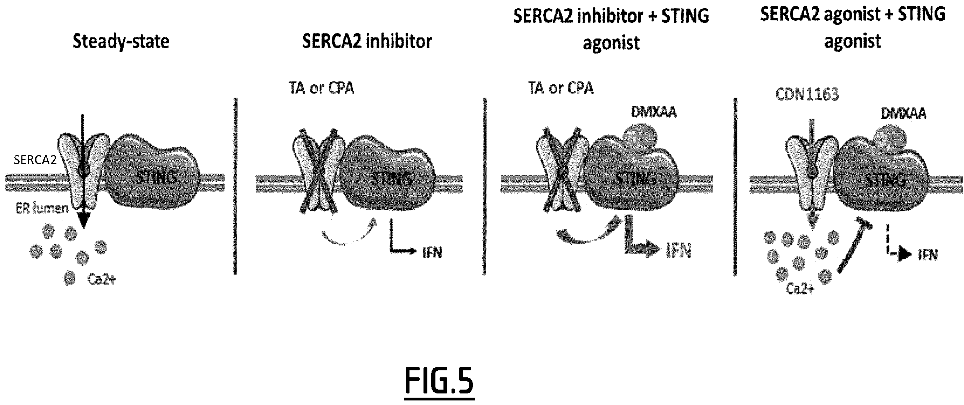

**Figure 5****: Impact of SERCA activity on the interferon response potentiation or inhibition.**

### Example: Modulation of SERCA2-dependent Calcium fluxes impacts the activation of STING-associated interferon responses

### MATERIALS AND METHODS:

### Cells and cell cultures

Wild type murine embryonic fibroblasts (WT-MEF) and MEF-Trex1^{-/-}, were maintained in Dulbecco's modified Eagle's medium (DMEM) supplemented with 10% fetal bovine serum (FBS), 1% penicillin/streptomycin and 1% glutamine.

WT-MEF were a gift of S. R. Paludan, MEFTrex1^{-/-} were obtained from J. Rehwinkel.

### Plasmids

shRNAs targeting ATP2A2 were obtained from Sigma-Aldrich (Clone ID:NM_009722.1-2015s1c1). shRNA-expressing lentiviral particles were produced by co-transfection of 2 × 10⁶ 293T cells with 5 µg of shSERCA2, 5 µg of psPAX2 GagPol, and 1 µg of p-MD2G envelope, using the standard calcium phosphate transfection protocol. Viral particles were harvested 24 hours after transfection, filtered with 0.45 µM filters, and used for transduction. For knockdown of SERCA2, 10⁶ MEF cells were seeded 24 hours before transduction. Medium was replaced 10 hours after transduction, and 1.5µg/ml puromycin selection was performed 72 hours later.

### RNA extraction and RT-qPCR

RNA was extracted using Trizol (Invitrogen) and treated with TURBO DNase (Ambion) according to manufacturer's protocols. Reverse transcription (SuperScript IV reverse transcriptase, Invitrogen) and qPCR using specific primers were performed using SYBR Green Master Mix (Takara) and LightCycler 480 cycler (Roche). mRNA levels were normalized to HSP90 mRNA levels.

### Whole-cell extract preparation and immunoblot

Cells were lysed in 30µl of TENTG-150 for 30 min at 4°C. Lysates were centrifuged 20 min at 12,000 rpm, and supernatants were collected for immunoblot. Protein quantification was performed using Bradford assay. Samples were resolved on SDS-PAGE, and proteins were transferred onto nitrocellulose membranes. Primary antibodies used include anti-phospho IRF3 (1:1000; Cell Signaling 4D4G), anti-IRF3 (1:1000; Cell Signaling D6l4C), anti-phospho STING S365 (1:1000; Cell Signaling D8F4W); anti-STING (1:1000; Cell Signaling D2P2F), anti-ATP2a2 (SERCA2) (1:1000, Cell Signaling D51B11); anti-HSP90 (1:1000; Cell Signaling C45G5); anti-phospho NPκβ p65 S536 (1:1000, Cell Signaling 93H1) and anti-NPκβ p65 (1:1000, Cell Signaling D14E12). All secondary antibodies (Cell Signaling) were used at 1:2000 dilution. Signal was visualized with SuperSignal West Pico Chemiluminescent Substrate (Thermo Fisher Scientific), and images were acquired on a ChemiDoc (Bio-Rad).

### Compounds (PubChem CID)

DMXAA (Vadimezan): 123964
CPA (Cyclopiazonic acid): 65261
Thapsigargin: 446378
CDN-1163: 16016585

### Oligonucleotide sequences

shATP2A2: 5'-CCGGCCAGGATTGAAGTAGCCTCTTCTCGAGAAGAGGCTACTTCAAT CCTGGTTTTT-3' (SEQ ID NO:1).
mlFNβ fwd 5'-CTGCGTTCCTGCTGTGCTTCTCCA-3' (SEQ ID NO:2)
mlFNβ rev 5'-TTCTCCGTCATCTCCATA GGGATC-3' (SEQ ID NO:3)
mCXCL10 fwd 5'-ATGACGGGCCAGTGAGAATG-3' (SEQ ID NO:4)
mCXCL10 rev 5'-TGAACACGTGGGCAGGATAG-3' (SEQ ID NO:5)
mlSG15 fwd 5'-GTGCTCCAGGACGGTCTTAC-3' (SEQ ID NO:6)
mlSG15 rev 5'-CTCGCTGCAGTTCTGTACCA-3' (SEQ ID NO:7)

### RESULTS:

### Inhibition of SERCA2 (ATP2A2) increased inflammatory response of WT-MEF and in chronic inflammation model Trex I KO MEF

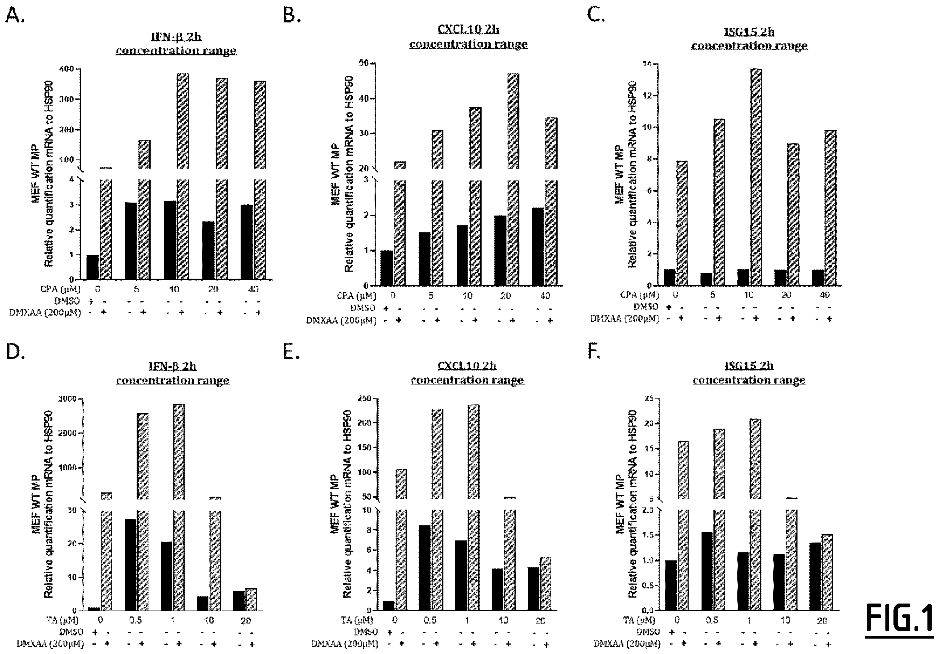

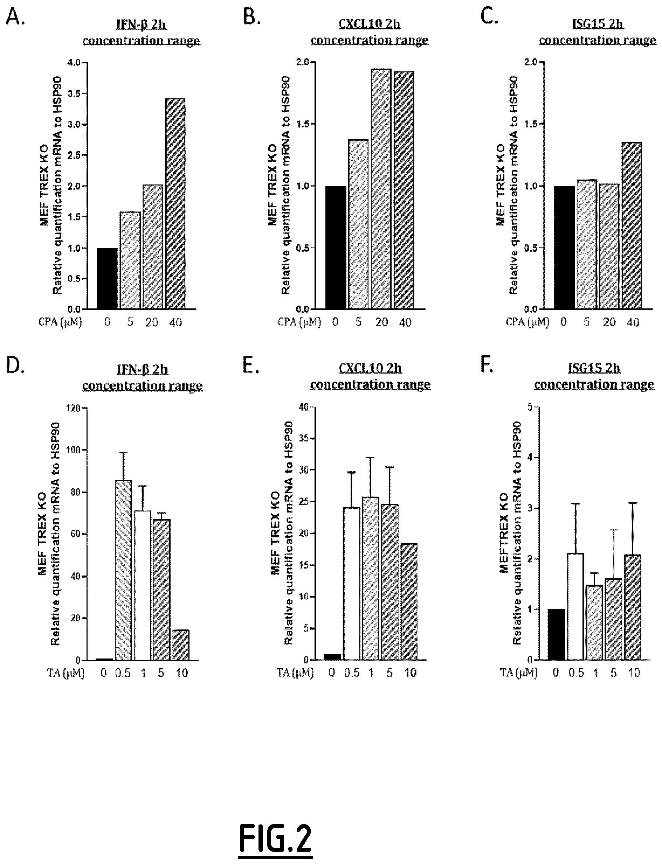

In order to investigate the impact of SERCA2 inhibition on inflammation associated with STING, two cellular models were used:
WT-MEF stimulated or not with DMXAA, a murine STING ligand which triggers STING-dependent inflammatory responses (Figure 1); and Trex I KO MEF which is a model of chronic STING activation (Figure 2).
Of note, Trex I deficiency is a model of type I interferonopathy.
WT-MEF or Trex I KO MEF were treated for 2h with SERCA2 inhibitors thapsigargin (TA) or CPA.
The inventors observed that treatment with SERCA2 inhibitors led to a potentiation of DMXAA-induced inflammation markers CXCL10 cytokine, Interferon Stimulated Gene (ISG-15) and Interferon-β (IFN-β) (Figure 1). Similarly, the inventors observed as well an increase of IFN-β, CXCL10 and ISG15 mRNA levels after CPA and TA treatment in Trex1-deficient cells (Figure 2).

Furthermore, the inventors confirmed that knocking-down SERCA2 before stimulation with DMXAA potentiates the expression of pro-inflammatory genes (Figure 3 A-C). In addition, they observed that SERCA2 knockdown leads to increased basal pIRF3 levels while upon DMXAA stimulation, an increase of pNF-κB protein is observed (Figure 3D). Thus, they show that inhibiting or decreasing SERCA2 protein levels potentiates both acute and chronic STING activation.

Because these data show that SERCA2 is an inhibitor of STING, the inventors next questioned whether SERCA2 activation would increase its inhibitory potential. To this aim, WT-MEFs were treated with the CDN-1163 SERCA2 agonist, and interferon response gene levels were assessed (Figure 4).
The first results show that under DMXAA stimulation, treatment with CDN-1163 inhibits IFN-β gene expression (Figure 4A).

### PERSPECTIVE:

The inventors propose a model where modulation of SERCA2 activity would modify anchorage of STING in the endoplasmic reticulum (ER) membrane (Figure 5). Inhibition of SERCA2 would induce a decrease of Ca²⁺ level in the ER and therefore release STING anchorage by STIM1. STING would be then more capable of being activated and translocated in the Golgi. In contrast, when SERCA2 is activated by its allosteric agonist, an increase in Ca²⁺ level in the ER would fortify STING anchorage by STIM1 and prevent its translocation in the Golgi.

Accordingly, these data demonstrate that SERCA2 inhibitors potentiate the interferon response both in chronic models of STING activation and upon acute stimulations. Based on these data, the invention proposes:
- the use of SERCA2 inhibitors, in the context of cancer or other pathology presenting a defective activation of STING and the interferon response. These could include viral pathologies where potentiation of the interferon response could allow faster elimination of the pathogen; and
- the use of SERCA2 agonists to decrease inflammation associated with STING. In addition, this could be of potential interest to chronic infectious diseases that include chronic STING activation, as well as to precancerous diseases in chronic inflammatory contexts.

## Claims

1. SERCA2 (Sarco/Endoplasmic Reticulum Ca²⁺-ATPase 2) modulator for use in treating an inflammation related-pathology.

2. SERCA2 modulator for use according to claim 1, wherein the SERCA2 modulator is a compound which binds to SERCA2 and is an inhibitor or an activator of SERCA2.

3. SERCA2 modulator for use according to claim 2, wherein the SERCA2 modulator is an inhibitor of SERCA2, and the inflammation-related pathology is a viral infection or cancer.

4. SERCA2 modulator for use according to claim 2 or 3, wherein the inhibitor of SERCA2 is a compound that reversibly or irreversibly binds to SERCA2 and decreases its activity, preferably the inhibitor is non-competitive, preferably the inhibitor is specific of SERCA2, preferably it is a small molecule.

5. SERCA2 modulator for use according to any one of claims 2 to 4, wherein the inhibitor of SERCA2 is thapsigargin or cyclopiazonic acid.

6. SERCA2 modulator for use according to claim 2, wherein the SERCA2 modulator is an activator of SERCA2, and the inflammation-related pathology is chosen from chronic infectious diseases that include chronic STING activation and cancer-related inflammation, preferably from chronic inflammatory diseases such as from lupus, Aicardi-Goutieres syndrome, obesity, inflammatory bowel disease (IBD) or arthritis; and cancer-related inflammation.

7. SERCA2 modulator for use according to claim 2 or 6, wherein the activator of SERCA2 is a compound that reversibly or irreversibly binds to SERCA2 and increases its activity, preferably the activator is allosteric, preferably it is a small molecule.

8. SERCA2 modulator for use according to any one of claims 2, 6 or 7, wherein the activator of SERCA2 is CDN-1163.

9. SERCA2 modulator for use according to any one of claims 3 to 8, wherein the cancer is solid or non-solid, and preferably selected from a colon cancer, a colorectal cancer, a melanoma, a bone cancer, a breast cancer, a thyroid cancer, a prostate cancer, an ovarian cancer, a lung cancer, a pancreatic cancer, a glioma, a cervical cancer, an endometrial cancer, a head and neck cancer, a liver cancer, a bladder cancer, a renal cancer, a skin cancer, a stomach cancer, a testis cancer, an urothelial cancer or an adrenocortical carcinoma, leukemia and lymphoma.

10. Products comprising:
a) at least one inhibitor of SERCA2 according to any one of claims 2 to 5, and
b) at least one activator of STING,
as combination products for a simultaneous, separate or sequential use in the treatment and/or prevention of a viral infection or a cancer.

11. Inhibitor of SERCA2 according to any one of claims 2 to 5, for use in preventing and/or treating a viral infection or a cancer in combination or in association with at least one activator of STING.

12. Inhibitor of SERCA2 according to any one of claims 2 to 5, for use in preventing and/or treating a viral infection or a cancer in a subject treated by at least one activator of STING.

13. Products according to claim 10 or inhibitor of SERCA2 for use according to claim 11 or 12, wherein the activator of STING is DMXAA, ADU-S100, amidobenzimidazole-based STING agonists and G10.


SEQUENCE LISTING

<110>  CNRS et al

<120>  SERCA modulators and their therapeutic uses

<130>  BET20P4422

<160>  7

<170>  PatentIn version 3.5

<210>  1
<211>  57
<212>  DNA
<213>  Artificial Sequence

<220>
<223>  shRNAs targeting ATP2A2

<400>  1
ccggccagga ttgaagtagc ctcttctcga gaagaggcta cttcaatcct ggttttt          57


<210>  2
<211>  24
<212>  DNA
<213>  Artificial Sequence

<220>
<223>  mIFNbeta forward

<400>  2
ctgcgttcct gctgtgcttc tcca                                              24


<210>  3
<211>  24
<212>  DNA
<213>  Artificial Sequence

<220>
<223>  mIFNbeta reverse

<400>  3
ttctccgtca tctccatagg gatc                                              24


<210>  4
<211>  20
<212>  DNA
<213>  Artificial Sequence

<220>
<223>  mCXCL10 forward

<400>  4
atgacgggcc agtgagaatg                                                   20


<210>  5
<211>  20
<212>  DNA

<213>  Artificial Sequence

<220>
<223>  mCXCL10 reverse

<400>  5
tgaacacgtg ggcaggatag                                                    20


<210>  6
<211>  20
<212>  DNA
<213>  Artificial Sequence

<220>
<223>  mISG15 forward

<400>  6
gtgctccagg acggtcttac                                                    20


<210>  7
<211>  20
<212>  DNA
<213>  Artificial Sequence

<220>
<223>  mISG15 reverse

<400>  7
ctcgctgcag ttctgtacca                                                    20